(19) Europäisches Patentamt European Patent Office Office européen des brevets

(11) **EP 1 161 965 B1**

(12) **FASCICULE DE BREVET EUROPEEN**

(45) Date de publication et mention
de la délivrance du brevet:
**08.08.2007 Bulletin 2007/32**

(51) Int Cl.:
***A61N 1/362*** *(2006.01)*

(21) Numéro de dépôt: **01401449.2**

(22) Date de dépôt: **07.06.2001**

(54) **Dispositif médical implantable actif tel que stimulateur cardiaque, défibrillateur, cardioverteur et/ou dispositif multisite à détection perfectionnée des extrasystoles auriculaires tardives**

Aktive implantierbare medizinische Vorrichtung, wie Herzschrittmacher, Entflimmerer und/oder Mehrstellen-Einrichtung mit verbesserter Erfassung von späten atrialen Extrasystolen

Active implantable medical device, of the type pacemaker, defibrillator, cardioverter and/or multisite device with improved detection of late atrial extrasystols

(84) Etats contractants désignés:
**AT BE CH CY DE DK ES FI FR GB GR IE IT LI LU MC NL PT SE TR**

(30) Priorité: **05.06.2000 FR 0007158**

(43) Date de publication de la demande:
**12.12.2001 Bulletin 2001/50**

(73) Titulaire: **ELA MEDICAL**
**92541 Montrouge (FR)**

(72) Inventeurs:
• **Poezevara, Yann**
**91080 Courcouronne (FR)**
• **Bonnet, Jean-Luc**
**45160 Olivet (FR)**

(74) Mandataire: **Dupuis-Latour, Dominique et al**
**SEP Pagenberg & Associés**
**14 boulevard Malesherbes**
**75008 Paris (FR)**

(56) Documents cités:
**EP-A- 0 885 626          WO-A-99/65564**

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs, ou encore les dispositifs "multisite", permettant de délivrer au coeur des impulsions électriques de faible énergie pour le traitement des troubles du rythme cardiaque.

Elle concerne plus précisément le traitement des troubles du rythme auriculaire par ces appareils.

Certains algorithmes de stimulation nécessitent à cet effet de bien discriminer les dépolarisations auriculaires sinusales d'avec les extrasystoles auriculaires (ESA), d'origine ectopique, avant d'engager des actions.

Les ESA sont en général caractérisées par un intervalle de couplage (c'est-à-dire un intervalle de temps séparant deux événements auriculaires successifs) relativement court.

Pour détecter ces ESA à intervalle de couplage court, les dispositifs connus utilisent des fenêtres ou périodes réfractaires déclenchées sur détection d'un événement (auriculaire ou ventriculaire, selon le cas), de sorte que toute activité auriculaire détectée à l'intérieur de cette fenêtre sera considérée comme une ESA par le logiciel du stimulateur, qui prendra alors des actions appropriées.

On peut ainsi prévoir :

- une fenêtre fixe déclenchée sur un événement ventriculaire, dite "PRAPV" (période réfractaire auriculaire post-ventriculaire), ou
- une fenêtre dynamique déclenchée sur un événement auriculaire ayant lui-même déclenché l'application d'un délai atrio-ventriculaire (DAV). Ce type de fenêtre sera désigné par la suite "PRRAD" (période réfractaire relative auriculaire dynamique).

Il arrive cependant que des ESA se produisent avec une faible prématurité, c'est-à-dire avec un intervalle de couplage relativement long.

Une telle ESA, qui sera appelée par la suite "ESA tardive", peut survenir après la fin de la PRAPV ou de la PRRAD et donc être vue par le stimulateur - à tort - comme une dépolarisation d'origine sinusale. Compte tenu de sa prématurité, cette ESA sera interprétée - ici encore à tort - comme une accélération du rythme sinusal du patient, ce qui va conduire l'algorithme de stimulation à prendre des actions inappropriées.

On peut prendre comme exemple l'algorithme dit d'"*overdriving* physiologique" tel que décrit notamment dans le EP-A-0 880 979 (ELA Médical) qui a pour but de stimuler en permanence les oreillettes après détection d'une ou plusieurs activations auriculaires d'origine sinusale. Le stimulateur augmente alors sa fréquence de stimulation de manière à couvrir le rythme spontané. Mais en cas d'ESA tardives, celles-ci vont produire une augmentation intempestive de la fréquence de stimulation, qui peut être mal tolérée par le patient et n'est en tout cas pas conforme à la physiologie de ce dernier.

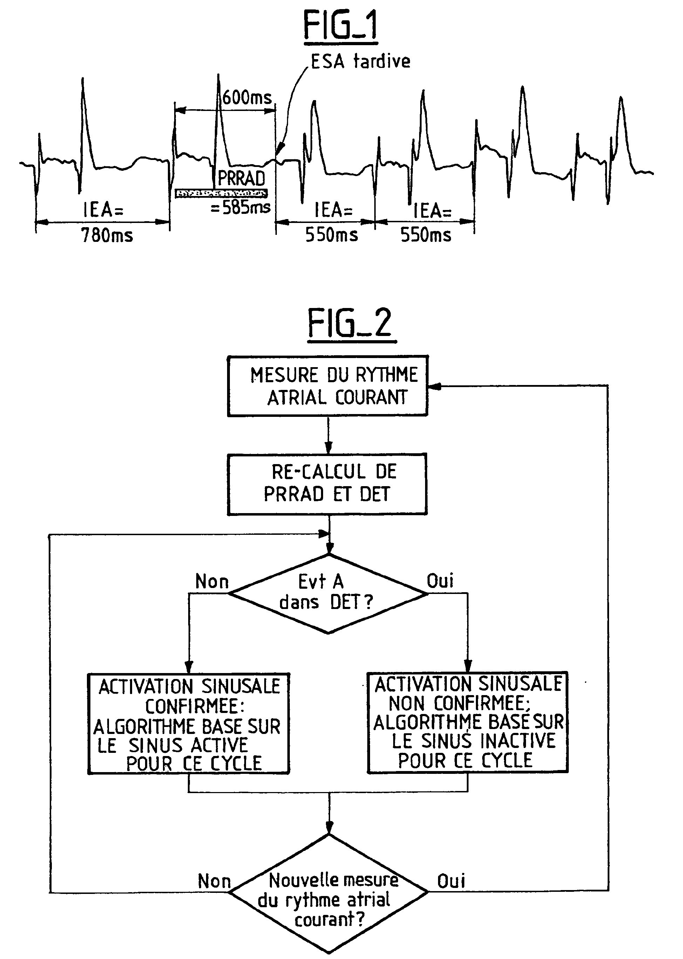

La figure 1 montre un tracé ECG correspondant à une telle situation, où un algorithme d'overdriving physiologique est perturbé par une ESA tardive.

À l'origine, l'intervalle d'échappement auriculaire IEA (c'est-à-dire l'intervalle de temps, compté après une détection ou une stimulation dans l'oreillette, à l'issue duquel une stimulation est délivrée à cette cavité si aucun événement spontané n'a été détecté) est de 780 ms, et l'algorithme a calculé sur cette base une durée de fenêtre dynamique PRRAD de 585 ms (75 % de 780 ms, correspondant à un taux de prématurité d'ESA de 25 %).

Si, comme illustré, une ESA tardive survient à 600 ms, c'est-à-dire avec une prématurité de 23 %, cette ESA tardive ne sera pas reconnue comme telle et sera vue par le stimulateur comme une dépolarisation auriculaire sinusale. La diminution de l'intervalle de couplage, de 780 à 600 ms, sera alors interprétée à tort comme une accélération du rythme sinusal, qui va provoquer une augmentation brusque de la fréquence de stimulation auriculaire, l'intervalle d'échappement passant de 780 à 550 ms.

L'augmentation de la durée des fenêtres PRAPV ou PRRAD ne pourrait pas remédier à cette difficulté, car un allongement excessif de ces dernières risquerait d'engendrer un nombre important de faux positifs, c'est-à-dire qu'en cas d'accélération sinusale spontanée du rythme auriculaire du patient, les dépolarisations sinusales seraient interprétées à tort comme des ESA, empêchant le stimulateur de réagir rapidement à l'accélération du rythme cardiaque.

Le WO-A-99/65564 décrit un stimulateur prévoyant d'appliquer aux événements auriculaires détachés deux périodes réfractaires auriculaires post-ventriculaires (PRAPV/PVARP) distinctes. Ces périodes réfractaires, déclenchées sur détection d'un événement ventriculaire, permettent de masquer les détections inopportunes d'extrasystoles ventriculaires.

L'un des buts de l'invention est de pallier la difficulté précitée liée à la survenue possible d'extrasystoles au niveau auriculaire (ESA), en différenciant les ESA tardives, d'origine ectopique, des accélérations sinusales spontanées du patient et éviter ainsi de leurrer les algorithmes du stimulateur par une fausse interprétation de la nature des dépolarisations auriculaires.

À cet effet, l'invention propose un dispositif du type connu par exemple d'après le WO-A-99/65564 précité, et comprenant les moyens énoncés dans la partie caractérisante de la revendication 1. Les sous-revendications visent des caractéristiques subsidiaires avantageuses.

On va maintenant décrire plus en détail la mise en oeuvre de l'invention, en référence aux dessins annexés.

La figure 1, précitée, est un électrocardiogramme montrant la manière dont un algorithme d'overdriving physiologique peut être perturbé par la survenue d'une ESA tardive.

La figure 2 est un organigramme général illustrant la manière dont est mise en oeuvre l'invention.

La figure 3 illustre une application de l'invention au cas d'une ESA tardive isolée.

La figure 4 illustre une application de l'invention au cas d'une accélération sinusale normale du rythme du patient.

Comme on l'a indiqué dans l'introduction de la présente description, les stimulateurs récents connus utilisent une fenêtre dynamique PRRAD permettant de filtrer certaines ESA.

Mais le rôle de cette fenêtre PRRAD est double :

- elle détecte certaines ESA, et
- elle assure la gestion du délai auriculo-ventriculaire (DAV) en évitant de synchroniser les ventricules sur des rythmes auriculaires rapides.

Il n'est pas possible d'accroître la durée de cette PRRAD sous peine, d'une part, d'augmenter le risque de détection de faux positifs et, d'autre part, de perturber gravement la gestion du DAV.

Le système proposé par l'invention est une fenêtre additionnelle, indépendante de la PRRAD, dont l'unique fonction est de repérer les ESA tardives.

Cette fenêtre sera par la suite dénommée "DET" (détection des extrasystoles tardives).

Cette fenêtre DET est complémentaire de la fenêtre PRRAD, mais ne la remplace pas.

La fenêtre DET est recalculée fréquemment, idéalement à chaque cycle, tout comme la PRRAD, afin de tenir compte du rythme atrial courant. Le calcul de la durée de cette fenêtre DET (voir plus bas) est tel que la fenêtre DET soit toujours plus longue que la fenêtre PRRAD - sous réserve que le paramètre *k* (voir plus bas) soit positif et que la fréquence atriale soit inférieure à un seuil donné .

Une détection auriculaire survenant dans la PRRAD sera toujours considérée comme une ESA.

En revanche, une détection auriculaire survenant dans la fenêtre DET ne sera pas toujours considérée comme une ESA.

En effet, cette détection peut soit provenir d'une accélération sinusale, soit être une ESA tardive. Dans le cas d'une telle détection, compte tenu de ce doute, le système de l'invention inhibera temporairement l'action des algorithmes dits "algorithmes basés sur le sinus", c'est-à-dire des algorithmes qui réagissent aux activations auriculaires sinusales, tels que les algorithmes cités d'overdriving physiologique.

L'analyse des événements suivants va permettre de lever l'incertitude sur la nature de l'événement auriculaire détecté :

- s'il s'agit d'une accélération sinusale, le nouveau rythme auriculaire va être soutenu et relativement stable d'un cycle à l'autre, de sorte que :

    - tant que la fenêtre DET n'a pas été recalculée, les activations auriculaires surviendront dans la fenêtre DET, et les algorithmes continueront d'être inhibés ;
    - dès que la fenêtre DET aura été recalculée à partir du rythme courant (conduisant donc à une valeur plus courte de la fenêtre), les détections auriculaires ne se situeront plus dans la fenêtre DET, mais après celle-ci ; les détections auriculaires suivantes pourront alors être considérées à juste titre comme des activations sinusales, et les algorithmes basés sur le sinus seront alors réactivés.

- dans le cas contraire, s'il s'agissait d'une ESA tardive et non d'une accélération sinusale, l'accélération du rythme auriculaire ne va pas être soutenue : il y aura donc un petit nombre de cycles avec détection auriculaire à l'intérieur de la fenêtre DET, cycles pendant lesquels les algorithmes basés sur le sinus seront inhibés. On évitera ainsi d'activer de façon inappropriée ces algorithmes.

L'organigramme de la figure 2 illustre l'enchaînement de ces diverses étapes.

Plus précisément, la durée de la fenêtre DET est calculée en fonction du rythme atrial courant (la mesure de ce rythme fait partie des fonctionnalités préexistantes du stimulateur). Cette fenêtre, tout comme la PRRAD, est initiée sur toute détection auriculaire pour laquelle le stimulateur déclenche un DAV.

Le rythme atrial courant est notamment déterminé par la durée IAM de l'intervalle auriculaire moyen ou "PP moyen", calculée par exemple sur huit cycles cardiaques ne comprenant pas d'ESA.

La fenêtre DET étant une fenêtre dynamique, évolutive en fonction du rythme atrial, ses performances seront améliorées si la mesure du rythme atrial courant est mise à jour souvent, en écartant les cycles anormaux. On peut prendre par exemple pour IAM la valeur de l'intervalle du dernier cycle auriculaire si celui-ci paraît normal (c'est-à-dire ne contient pas d'événement prématuré ou d'interférence), dans le cas contraire on prendra la moyenne des *n* derniers cycles normaux (par exemple *n* = 8).

La durée, en millisecondes, de la fenêtre DET sera avantageusement calculée par :

$$DET = a \times IAM + k.$$

Le coefficient *a* (*a* < 1), permet d'obtenir une fenêtre DET plus courte que le rythme atrial courant (sinon tous les événements surviendraient à l'intérieur de la fenêtre). Une valeur *a* = 0,625 permet par exemple de détecter les ESA ayant une prématurité d'au moins 37,5 %.

L'utilisation de la constante *k* est optionnelle. Cette constante, par exemple *k* = 175 ms, allonge la valeur de DET de manière qu'au-delà d'un certain niveau de fréquence cardiaque les détections auriculaires se produisent systématiquement à l'intérieur de la fenêtre DET. Ceci permet d'inhiber en toutes circonstances (et non plus conditionnellement) les algorithmes basés par le sinus, notamment les algorithmes d'overdriving physiologique, chaque fois que la fréquence atriale s'élève au-dessus d'un seuil prédéterminé. Dans certaines configurations, le paramètre *k* peut être choisi négatif ; dans ce dernier cas, il n'y aura pas de limitation au-delà d'une fréquence seuil.

En revenant à la figure 2, une fois la durée de la fenêtre DET recalculée, sur chaque détection d'un événement auriculaire EvtA on compare l'intervalle de couplage de cet événement à la valeur de la fenêtre DET. Si la détection survient à l'intérieur de la fenêtre DET, alors l'algorithme basé sur le sinus est inactivé pour (au moins) la durée de ce cycle. Dans le cas contraire, l'algorithme est activé normalement.

Les détections suivantes sont traitées de la même façon, et la fenêtre DET est recalculée en fonction du rythme atrial courant dès que celui-ci est mis à jour.

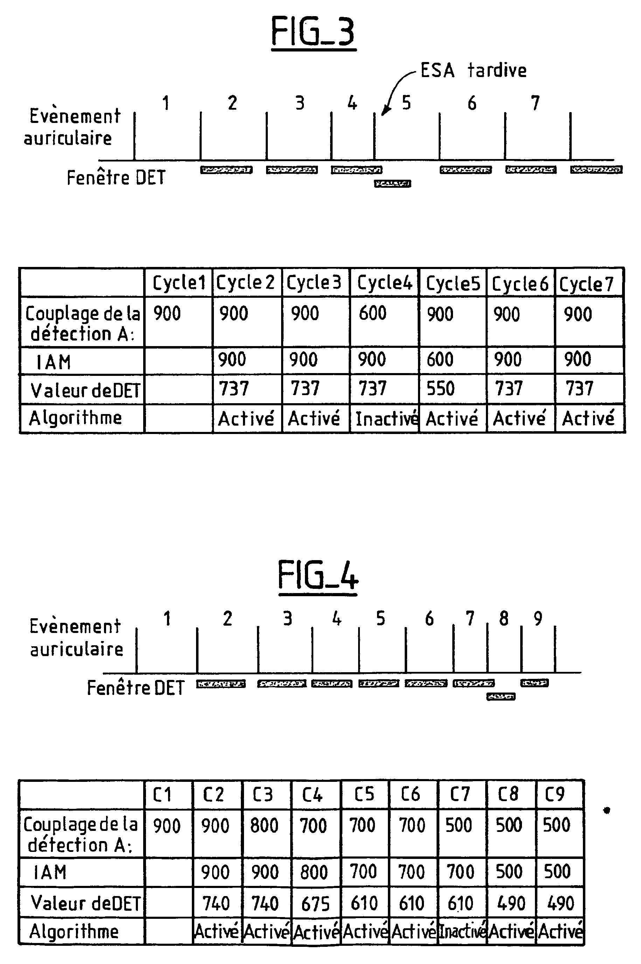

Les figures 3 et 4 illustrent deux applications de l'invention, respectivement dans le cas d'une ESA tardive isolée et dans celui d'une accélération sinusale normale.

Sur chacune de ces figures, on a présenté un chronogramme avec les événements auriculaires successifs et les fenêtres DET calculées correspondantes, ainsi qu'un tableau donnant, pour chaque cycle, les valeurs de l'intervalle de couplage, de l'intervalle auriculaire moyen, de la durée de la fenêtre DET et l'état, activé ou inactivé, d'un algorithme basé sur le sinus.

On supposera que la durée de la fenêtre DET est calculée conformément à l'expression DET = 0,625 x IAM + 175, et que la valeur de IAM est mise à jour à chaque cycle.

Dans le cas de la figure 3, le quatrième cycle illustré se termine par une ESA tardive, avec un intervalle de couplage de 600 ms. La durée de la fenêtre DET calculée pour le rythme courant (intervalle de couplage normal constant de 900 ms) étant de 737 ms avec les données numériques indiquées plus haut, l'ESA survient à l'intérieur de la fenêtre DET, ce qui a pour effet d'inhiber l'algorithme basé sur le sinus.

Au cycle suivant (n° 5), l'intervalle de couplage ayant retrouvé la valeur (900 ms) qu'il avait avant, l'événement auriculaire survient hors de la fenêtre DET (recalculée à 550 ms), ce qui a pour effet de réactiver l'algorithme, restaurant ainsi le fonctionnement du stimulateur à son état normal antérieur.

Dans le cas de la figure 4, en revanche, la fréquence auriculaire augmente régulièrement en raison d'une accélération sinusale, normale.

Pendant les six premiers cycles, la valeur de la fenêtre DET s'adapte à cette accélération, et l'algorithme basé sur le sinus reste activé.

Au cours du septième cycle, l'accélération est un peu plus forte, conduisant à une diminution rapide de l'intervalle de couplage. Le huitième événement auriculaire détecté (intervalle de couplage 500 ms) survient alors à l'intérieur de la fenêtre DET (durée 610 ms) et, par sécurité, l'algorithme basé sur le sinus est inactivé.

L'analyse de l'événement auriculaire suivant, qui révèle le maintien du rythme soutenu, permet de lever le doute en écartant l'hypothèse d'une ESA tardive. La fenêtre DET ayant été réduite (de 610 à 490 ms), ce nouvel événement, qui présente par rapport au précédent un intervalle de couplage maintenu à 500 ms, survient maintenant hors de la fenêtre DET, ce qui a pour effet de réactiver l'algorithme, qui pourra prendre si nécessaire les actions appropriées correspondant à l'accélération sinusale.

## Revendications

1. Un dispositif médical implantable actif, tel qu'un stimulateur cardiaque, défibrillateur, cardioverteur et/ou dispositif multisite, permettant de délivrer au coeur des impulsions électriques de faible énergie pour le traitement des troubles du rythme cardiaque, comprenant :

   - des moyens de détection d'événements cardiaques auriculaires (EvtA),
   - des moyens pour appliquer aux événements auriculaires détectés une première fenêtre formant période réfractaire relative auriculaire,
   - des moyens pour appliquer aux événements auriculaires détectés une seconde fenêtre formant période réfractaire auriculaire, distincte de la première fenêtre et de durée supérieure, et
   - des moyens réagissant à une variation du rythme auriculaire sinusal détecté et filtré par ladite première fenêtre,

   **caractérisé :**

   - **en ce que** lesdites première fenêtre (PRRAD) et seconde fenêtre (DET) sont des fenêtres déclenchées en réponse à un événement auriculaire, pour l'élimination des extrasystoles auriculaires,
   - **en ce que**, pour l'élimination des extrasystoles auriculaires à faible prématurité, la durée de la seconde fenêtre est une durée variable définie comme une fraction de l'intervalle auriculaire moyen courant (IAM),

- et **en ce qu'**il comprend en outre des moyens pour inhiber temporairement lesdits moyens réagissant à une variation du rythme auriculaire sinusal, en cas de détection d'un événement auriculaire à l'intérieur de la seconde fenêtre.

**2.** Le dispositif de la revendication 1, dans lequel la durée DET de la se conde fenêtre est donnée par l'expression :

$$DET = a \times IAM + k,$$

avec $0 < a < 1$, $a$ et $k$ étant choisis de manière que la durée de la seconde fenêtre soit inférieure à celle de l'intervalle auriculaire moyen courant IAM.

**3.** Le dispositif de la revendication 2, dans lequel $a$ = 0,625.

**4.** Le dispositif de la revendication 2, dans lequel $k$ est positif et choisi de manière que les détections auriculaires se produisent systématiquement à l'intérieur de la seconde fenêtre au-delà d'un niveau prédéterminé de fréquence cardiaque.

**5.** Le dispositif de la revendication 4, dans lequel $k$ = 175 ms.

**6.** Le dispositif de la revendication 1, dans lequel la première fenêtre est une fenêtre dynamique (PRRAD) déclenchée sur un événement auriculaire ayant lui-même déclenché l'application d'un délai atrio-ventriculaire (DAV).

**Claims**

**1.** An active implantable medical device, such as a pacemaker, defibrillator, cardioverter and/or multisite device, able to deliver low energy electric pulses to the heart for the treatment of heart rate disorders, comprising:

 - means for detecting atrial cardiac events (EvtA),
 - means for applying a first window forming an atrial relative refractory period to the detected atrial events,
 - means for applying a second window forming an atrial refractory period, distinct from the first window and of longer duration, to the detected atrial events, and
 - means reacting to a variation of the atrial sinus rhythm detected and filtered by said first window,

**characterised:**

 - **in that** said first window (PRRAD) and second window (DET) are windows, which are triggered in response to an atrial event to eliminate the atrial extrasystoles,
 - **in that**, in order to eliminate the slightly premature atrial extrasystoles, the duration of the second window is a variable duration defined as a fraction of the current mean atrial interval (IAM),
 - and **in that** it further comprises means for temporarily inhibiting said means reacting to a variation of the atrial sinus rhythm in case of detection of an atrial event inside the second window.

**2.** The device of claim 1, wherein the duration DET of the second window is given by the expression:

$$DET = a \times IAM + k,$$

with $0<a<1$, a and k being selected so that the duration of the second window is less than the one of the current mean atrial interval IAM.

**3.** The device of claim 2, wherein a = 0.625.

**4.** The device of claim 2, wherein k is a positive value and selected so that the atrial detections occur systematically inside the second window beyond a predetermined heart rate level.

**5.** The device of claim 4, wherein k = 175 ms.

**6.** The device of claim 1, wherein the first window is a dynamic window (PRRAD) triggered upon an atrial event having itself triggered the application of an atrioventricular delay (AVD).

**Patentansprüche**

**1.** Aktive implantierbare medizinische Vorrichtung, wie z.B. Herzschrittmacher, Defibrillator, Kardioverter und/oder Multisite-Vorrichtung, die es erlaubt, an das Herz elektrische Impulse geringer Energie zur Behandlung von Herzrhythmusstörungen abzugeben, mit:

 - Mitteln zur Erfassung von Herzvorhofereignissen (EvtA),
 - Mitteln zur Anwendung eines ersten Fensters, das eine relative atriale refraktäre Periode bildet, auf die erfassten Herzvorhofereignisse,
 - Mitteln zur Anwendung eines zweiten Fensters, das eine refraktäre atriale Periode bildet, die sich vom ersten Fenster unterscheidet und von längerer Dauer ist, auf die erfassten Herzvorhofereignisse, und
- Mitteln, die auf eine Veränderung des atrialen Sinusrhythmus reagieren, der durch das erste Fenster erfasst und gefiltert wird,

**dadurch gekennzeichnet:**

- **dass** das erste Fenster (PRRAD) und zweite Fenster (DET) Fenster sind, die in Antwort auf ein Herzvorhofereignis ausgelöst werden, zur Beseitigung der atrialen Extrasystolen,
- **dass** zur Beseitigung der leicht verfrühten atrialen Extrasystolen die Dauer des zweiten Fensters eine variable Dauer ist, die als ein Bruchteil des gegenwärtigen mittleren atrialen Intervalls (IAM) definiert ist,
- und **dass** sie außerdem Mittel umfasst, um im Falle der Erfassung eines Herzvorhofereignisses innerhalb des zweiten Fensters vorübergehend die Mittel zu unterdrücken, die auf eine Veränderung des atrialen Sinusrhythmus reagieren.

2. Vorrichtung nach Anspruch 1, bei welcher die Dauer DET des zweiten Fensters durch den folgenden Ausdruck gegeben ist:

$$DET = a \times IAM + k,$$

mit $0 < a < 1$, wobei a und k so gewählt sind, dass die Dauer des zweiten Fensters geringer als diejenige des gegenwärtigen mittleren atrialen Intervalls IAM ist.

3. Vorrichtung nach Anspruch 2, bei welcher a = 0,625.

4. Vorrichtung nach Anspruch 2, bei welcher k positiv ist und so gewählt ist, dass die Herzvorhoferfassungen oberhalb eines vorbestimmten Pegels der Herzfrequenz systematisch innerhalb des zweiten Fensters erfolgen.

5. Vorrichtung nach Anspruch 4, bei welcher k = 175 ms.

6. Vorrichtung nach Anspruch 1, bei welcher das erste Fenster ein dynamisches Fenster (PRRAD) ist, das bei einem Herzvorhofereignis ausgelöst wird, welches selber die Anwendung einer atrioventrikulären Verzögerung (AVV) ausgelöst hat.

## FIG_1

## FIG_2

# FIG_3

ESA tardive

Evènement auriculaire

|                              | Cycle 1 | Cycle 2 | Cycle 3 | Cycle 4 | Cycle 5 | Cycle 6 | Cycle 7 |
|------------------------------|---------|---------|---------|---------|---------|---------|---------|
| Couplage de la détection A:  | 900     | 900     | 900     | 600     | 900     | 900     | 900     |
| I AM                         |         | 900     | 900     | 900     | 600     | 900     | 900     |
| Valeur de DET                |         | 737     | 737     | 737     | 550     | 737     | 737     |
| Algorithme                   |         | Activé  | Activé  | Inactivé| Activé  | Activé  | Activé  |

# FIG_4

Evènement auriculaire

Fenêtre DET

|                              | C1  | C2   | C3   | C4   | C5   | C6   | C7      | C8   | C9   |
|------------------------------|-----|------|------|------|------|------|---------|------|------|
| Couplage de la détection A:  | 900 | 900  | 800  | 700  | 700  | 700  | 500     | 500  | 500  |
| I AM                         |     | 900  | 900  | 800  | 700  | 700  | 700     | 500  | 500  |
| Valeur de DET                |     | 740  | 740  | 675  | 610  | 610  | 610     | 490  | 490  |
| Algorithme                   |     | Activé| Activé| Activé| Activé| Activé| Inactivé| Activé| Activé|

**EP 1 161 965 B1**

**RÉFÉRENCES CITÉES DANS LA DESCRIPTION**

**Documents brevets cités dans la description**

- EP 0880979 A
- WO 9965564 A